# EUROPÄISCHE PATENTSCHRIFT

(11) **EP 1 218 519 B1**
(45) Veröffentlichungstag und Bekanntmachung des Hinweises auf die Patenterteilung: **27.06.2007**
(21) Anmeldenummer: 00951443.1
(22) Anmeldetag: 25.07.2000
(51) Int. Cl.: C12N 15/55, C12N 9/18, C12N 5/12, C07K 16/40, C12P 7/64

(54) **ESTERGRUPPENSPALTENDES ENZYM AUS THERMOMONOSPORA FUSCA**
ENZYME WHICH CLEAVES ESTER GROUPS AND WHICH IS DERIVED FROM THERMOMONOSPORA FUSCA
ENZYME DE CLIVAGE DE GROUPES ESTER A PARTIR DE THERMOMONOSPORA FUSCA

(30) Priorität: 30.09.1999 DE 19947286
(43) Veröffentlichungstag der Anmeldung: 03.07.2002
(73) Patentinhaber: Gesellschaft für Biotechnologische Forschung mbH (GBF), 38124 Braunschweig (DE)
(72) Erfinder: DECKWER, Wolf-Dieter, D-38124 Braunschweig (DE); MÜLLER, Rolf-Joachim, D-38124 Braunschweig (DE); KLEEBERG, Ilona, D-38124 Braunschweig (DE); VAN DEN HEUVEL, Joop, D-38124 Braunschweig (DE)
(74) Vertreter: Boeters, Hans Dietrich
(86) Internationale Anmeldenummer: PCT/EP2000/007115
(87) Internationale Veröffentlichungsnummer: WO 2001/023581

(56) Entgegenhaltungen:
- WO-A-95/25707
- DE-A- 19 706 023
- BACHMANN S L ET AL: "PURIFICATION AND COOPERATIVE ACTIVITY OF ENZYMES CONSTITUTING THE XYLAN-DEGRADING SYSTEM OF THERMOMONOSPORA-FUSCA" APPLIED AND ENVIRONMENTAL MICROBIOLOGY, Bd. 57, Nr. 8, 1991, Seiten 2121-2130, XP000979271 ISSN: 0099-2240
- HOLLICK G E: "ENZYMATIC PROFILES OF SELECTED THERMOPHILIC ACTINOMYCETES" MICROBIOS, Bd. 35, Nr. 141-142, 1982, Seiten 187-196, XP000979248 ISSN: 0026-2633
- MCCARTHY A J ET AL: "Xylan-degrading enzymes produced by the thermophilic actinomycete Thermomonospora fusca" PROG.BIOTECHNOL., 1992, Bd. 7, 1992, Seiten 309-13, XP000979410
- CRUZ HUGO ET AL: "Sequence of the Streptomyces albus G lipase-encoding gene reveals the presence of a prokaryotic lipase family." GENE (AMSTERDAM), Bd. 144, Nr. 1, 1994, Seiten 141-142, XP002159117 ISSN: 0378-1119 in der Anmeldung erwähnt
- PEREZ CRISTINA ET AL: "Cloning, characterization, and expression in Streptomyces lividans 66 of an extracellular lipase-encoding gene from Streptomyces sp. M11." GENE (AMSTERDAM), Bd. 123, Nr. 1, 1993, Seiten 109-114, XP002159118 ISSN: 0378-1119 in der Anmeldung erwähnt
- KEMPF, ALEXANDER ET AL: "Screening of thermophilic actinomycetes for biopolymer degrading enzymes" DECHEMA BIOTECHNOL. CONF. (1989), 3(PT. A, JT. MEET. SIM DECHEMA, PRESENTATION BIOCHEM. LAB., MICROB. PRINCK.BIOPROCESSES, APPL. GENET.), 159-62 , XP000979280

### SEQUENZPROTOKOLL as ANNEX

<110> Gesellschaft fuer Biotechnologische Forschung mbH
<120> Estergruppenspaltendes Enzym aus Thermomonoapora fusca
<130> PCT/EP00/07115
<140> PCT/EPOO/07115
   <141> 2000-07-25
<150> DE 199 47 286.6
   <151> 1999-09-30
<160> 1
<170> PatentIn Ver. 2.1
<210> 1
   <211> 261
   <212> PRT
   <213> Thermomonospora fusca
<400> 1
   Ala Asn Pro Tyr Glu Arg Gly Pro Asn Pro Thr Asp Ala Leu Leu Glu
    1               5                  10                  15
   Ala Ser Ser Gly Pro Phe Ser Val Ser Glu Glu Asn Val Ser Arg Leu
               20                  25                  30
   Ser Ala Ser Gly Phe Gly Gly Gly Thr Ile Tyr Tyr Pro Arg Glu Asn
           35                  40                  45
   Asn Thr Tyr Gly Ala Val Ala Ile Ser Pro Gly Tyr Thr Gly Thr Glu
       50                  55                  60
   Ala Ser Ile Ala Trp Leu Gly Glu Arg Ile Ala Ser His Gly Phe Val
   65                  70                  75                  80
   Val Ile Thr Ile Asp Thr Ile Thr Thr Leu Asp Gln Pro Asp Ser Arg
                   85                  90                  95
   Ala Glu Gln Leu Asn Ala Ala Leu Asn His Met Ile Asn Arg Ala Ser
               100                 105                 110
   Ser Thr Val Arg Ser Arg Ile Asp Ser Ser Arg Leu Ala Val Met Gly
           115                 120                 125
   His Ser Met Gly Gly Gly Gly Thr Leu Arg Leu Ala Ser Gln Arg Pro
       130                 135                 140
   Asp Leu Lys Ala Ala Ile Pro Leu Thr Pro Trp His Leu Asn Lys Asn
   145                 150                 155                 160
   Trp Ser Ser Val Thr Val Pro Thr Leu Ile Ile Gly Ala Asp Leu Asp
                   165                 170                 175
   Thr Ile Ala Pro Val Ala Thr His Ala Lys Pro Phe Tyr Asn Ser Leu
               180                 185                 190
   Pro Ser Ser Ile Ser Lys Ala Tyr Leu Glu Leu Asp Gly Ala Thr His
           195                 200                 205
   Phe Ala Pro Asn Ile Pro Asn Lys Ile Ile Gly Lys Tyr Ser Val Ala
       210                 215                 220
   Trp Leu Lys Arg Phe Val Asp Asn Asp Thr Arg Tyr Thr Gln Phe Leu
   225                 230                 235                 240
   Cys Pro Gly Pro Arg Asp Gly Leu Phe Gly Glu Val Glu Glu Tyr Arg
                   245                 250                 255
   Ser Thr Cys Pro Phe
               260

## Beschreibung

Die Erfindung betrifft ein Estergruppen von Polyestern spaltendes Enzym (im folgenden auch EGS-Enzym genannt) aus Thermomonospora fusca, ein Verfahren zu dessen Herstellung sowie seine Verwendung zum Abbau bzw. zur Behandlung von Estergruppen enthaltenden Polymeren. Das Enzym eignet sich auch zum Spalten von niedermolekularen Verbindungen.

### Einleitung und Stand der Technik

Polymere und makromolekulare Werkstoffe, die einem kontrollierten biologischen Abbau unterliegen können, gewinnen in den letzten Jahren zunehmend an Bedeutung. Eine Reihe von derartigen Produkten sind auf dem Markt bereits im industriellen Maßstab verfügbar. Innerhalb dieser neuartigen Produkte nehmen Estergruppen enthaltende Polymere (z.B. Polyester, Polyesterurethane, Polyesteramide) eine zentrale Rolle ein. Beispiele für bioabbaubare Kunststoffe auf Polyesterbasis sind z.B. Poly(β-hydroxybutyrat-co-β-hydroxyvalerat), Poly(ε-caprolacton) oder Poly(butylensuccinat).

Da Polymere aufgrund ihrer Molekülgröße die äußere Membran der mikrobiellen Zellen nicht passieren können, ist der erste und in der Regel geschwindigkeitsbestimmende Schritt des Abbaus eine Molmassenreduzierung (Depolymerisierung) durch extrazelluläre Enzyme. Polyester sind deshalb potentiell bioabbaubar, da die Esterbindungen grundsätzliche Angriffspunkte für solche extrazellulären hydrolysierenden Enzyme darstellen

Für aliphatische Polyester sind seit langem Untersuchungen zum biologischen Abbau mit Hilfe solcher hydrolysierenden Enzyme (z.B. Lipasen, PHB-Depolymerasen) bekannt [Tokiwa et al., Polym. Mater. Sci. Eng. 62(1990), 988-992] [Jendrossek et al., Appl. Microbiol. Biotechnol. 46(1996), 451-4631]. Das Material wird mit einem entsprechenden Enzym unter geeigneten Bedingungen inkubiert und der Abbau über die Bildung von Spaltprodukten im umgebenden Medium oder über den Gewichtsverlust der Proben bestimmt. Für die natürlichen Polyhydroxyalkanoate wurden in der Regel hierfür speziell isolierte Hydrolysen (PHB-Depolymerasen) eingesetzt, während für den Abbau synthetischer Polyester nicht speziell für den Zweck des Polymerabbaus isolierte kommerzielle Lipasen etc. verwendet wurden.

Während viele aliphatische Polyester sich grundsätzlich als biologisch angreifbar erwiesen haben, gelten aromatische Polyester [z.B. Poly(ethylenterephthalat), Poly(propylenterephthalat), Polybutylenterephthalat)] bekanntermaßen als biologisch resistent. Um die vergleichsweise zu aliphatischen Polyestern besseren Verarbeitungs- und Anwendungseigenschaften der aromatischen Strukturen zu nutzen, sind in den letzten Jahren biologisch abbaubare aliphatische-aromatische Copolyester entwickelt worden und werden in industriellem Maßstab hergestellt [Presseinformation der BASF AG, Ludwigshafen, zur K'98-Messe in Düsseldorf vom 17.03.98].

Durch die Einführung der aromatischen Komponenten wird jedoch die biologische Abbaugeschwindigkeit signifikant vermindert [Müller et al., Polym. Degrad. Stab. 59 (1998), S. 203-208]. So kommen z.B. Jun et al. [Jun et al., J. Environ., Polym. Degrad. 2(1) (1994), S. 9-18] zu dem Schluß, daß Copolyester aus PET und PCL nicht signifikant durch Lipasen (z.B. Pseudomonas-sp.-Lipase) angegriffen werden.

Ein Abbau von insbesondere Polyesteramiden mit verschiedenen üblichen kommerziellen Lipasen unter technischen Aspekten ist kürzlich beschrieben worden [WO 98/36086]. In diesem Patent wird auch die Auflösung eines Copolyesters aus Butandiol, Terephthalat (40 Mol.-%) und Adipat (60 Mol.-%) beschrieben. Die vermeintlich für technische Anwendungen geeignete Reaktionen werden durch beispielweise 50 mg Enzym (Lipase aus Candida antarctica) zu 0,3-1,8 g eines Polyesteramides in Folien- bzw. Plattenform erreicht. Die erzielten Abbauraten liegen im Bereich von 600 mg Abbau/Woche. Für den beschriebenen Abbau des aliphatisch-aromatischen Copolyesters muß eine Enzymmenge von 1% (in 100 ml Puffer) zu einem feinen Pulver des Copolyesters gegeben werden. Trotz der durch die kleine Partikelgröße bedingten erheblich größeren Oberfläche wird hier nur ein Abbau von 230 mg/Woche erreicht.

Kürzlich konnte gezeigt werden, daß aliphatisch-aromatische Copolyester durch Mikroorganismenstämme aus der Gruppe der Actinomyceten abgebaut werden können [Kleeberg et al., Appl. Environ. Polym. Degrad. 64(5) (1998), 1731-1735].

Trotzdem besteht immer noch ein Bedarf nach einem hochaktiven estergruppenspaltenden Enzym, daß Polymere auf Polyesterbasis abbauen kann.

Überraschenderweise wurde erfindungsgemäß gefunden, daß biologisch abbaubare, Polyestergruppen enthaltende Polymere, insbesondere auch aliphatisch-aromatische Copolyester, mit dem erfindungsgemäßen, im folgenden näher spezifizierten, extrazellulären Enzym aus dem zu den Actinomyceten gehörenden Mikroorganismus Thermomonospora fusca, insbesonere des Stammes Thermomonospora fusca DSM 43793, alleine oder im Gemisch mit anderen Enzymen mit einer außergewöhnlich hohen Abbaugeschwindigkeit bzw. -rate depolymerisiert und in niedermolekulare Bruchstücke zerlegt werden können.

Die Erfindung betrifft somit ein Estergruppen von Polyestern spaltendes Enzym nach Patentanspruch 1, ein synthetisches estergruppenspaltendes Protein nach Patentanspruch 5, polyklonale bzw. monoklonale Antikörper nach Patentanspruch 6, bzw. 7, Hybridomzellen nach Patentanspruch 8, eine estergruppenspaltende Zusammensetzung nach Patentanspruch 9 sowie die Verwendung eines estergruppenspaltenden Enzyms, synthetischen Proteins oder einer estergruppenspaltenden Zusammensetzung nach Patenanspruch 12.

Vorteilhafte Ausführungsformen sind Gegenstand der Unteransprüche.

Konkreter, jedoch ohne Einschränkung, betrifft die Erfindung ein Estergruppen von Polyestern spaltendes Enzym, das erhältlich ist, indem der Mikroorganismus Thermomonospora fusca in einem geeigneten Nährmedium in Anwesenheit eines Induktors kultiviert wird, aus dem Nährmedium ein Überstand mit einem Gehalt an einem Estergruppen von Polyestern spaltenden Enzym gewonnen wird, und das Enzym mit üblichen biochemischen Reinigungsmethoden gereinigt wird, wobei das Enzym durch folgende Parameter gekennzeichnet ist:
Molmasse: 27400 D (durch SDS-Gelelektrophorese bestimmt) bzw. 28200 D (aus der Aminosäuresequenz berechnet),
Temperaturoptimum/-bereich: 65°C (30-80°C),
Temperaturstabilität 70°C/30 min.,
pH-Optimum/-bereich: 6-7 (4- >8),
isoelektrischer Punkt: 6,4.

Vorzugsweise stammt das erfindungsgemäße estexgxuppenspaltende Enzym aus dem Thermomonospora-fusca-Stamm, der bei der Deutschen Sammlung für Mikroorganismen unter der Zugangsnummer DSM 43793 hinterlegt ist.

Die Kultivierung kann durch im Batch-, Fed-Batch- oder kontinuierlichen Betrieb in synthetischen oder komplexen Medien erfolgen. Die Mikroorganismen können dabei frei vorliegen oder an einem festen Träger immobilisiert sein. Grundsätzlich kommen sowohl natürliche als auch genetisch veränderte Mikroorganismen in Frage.

Geeignete Induktoren für die Ausscheidung des Enzyms sind beispielsweise die Substrate selber, z.B. aliphatische Polyester und/oder Oligoester, aliphatisch-aromatische Copolyester.

In einer bevorzugten Ausführungsform wird das erfindungsgemäße estergruppenspaltende Enzym außerdem aus dem Nährmedium isoliert, indem aus dem Nährmedium ein enzymhaltiger Kulturüberstand gewonnen wird, beispielsweise durch Zentrifugation, der gegebenenfalls konzentriert werden kann, beispielsweise durch Ultrafiltration und/oder Ammoniumsulfatfällung, worauf mit üblichen biochemischen Reinigungsmethoden, beispielsweise durch Chromatographie, insbesondere durch Ionenaustausch- und/oder hydrophobe Interaktionschromatographie, das Enzym gereinigt wird.

Das erfindungsgemäße estergruppenspaltende Enzym aus Thermomonospora fusca DSM 43793 ist durch folgende Parameter gekennzeichnet:
Molmasse: 27400 D (durch SDS-Gelelektrophorese bestimmt) bzw. 28200 D'(aus der Aminosäuresequenz berechnet)
Temperaturoptimum/-bereich: 65°C (30-80°C),
Temperaturstabilität: 70°C/30 min,
pH-Optimum/-bereich: 6-7 (4- >8),
Isoelektrischen Punkt: 6,4.

Die Substratspezifität umfaßt Estergruppen enthaltende Polymere, Triglyceride, Phthalsäureester.

Nach einer bevorzugten Ausführungsform hat das erfindungsgemäße estergruppenspaltende Enzym aus Thermomonospora DSM 43793 die folgende Aminosäuresequenz: oder
durch eine durch Substitution, Insertion oder Deletion von Aminosäuren entstandene mutierte Aminosäuresequenz, die ein isofunktionelles Enzym ergibt.

Die obige Aminosäuresequenz kann selbstverständlich auch synthetisch nach herkömmlichen Verfahren hergestellt werden, beispielsweise mit einem automatischen "Peptide-Synthesizer".

Die Erfindung betrifft ferner polyklonale und monoklonale Antikörper, die spezifisch gegen ein erfindungsgemäßes esterspaltendes Enzym oder gegen ein entsprechendes synthetisches Protein mit gleicher Funktion und/oder Aminosäuresequenz gerichtet sind, sowie Hybridomzellen, welche die monoklonalen Antikörper bilden. Die Herstellung von poly- oder monoklonalen Antikörpern bzw. die Herstellung der die letzteren bildenden Hybridome ist seit langem bekannt (vgl. beispielsweise: E. Harlow, D. Lane, "Antibodies, A Laboratory Manual", Cold Spring Harbor Laboratory, 1988; E. Lidell, I. Weeks, "Antikörper-Techniken", Spektrum Akademischer Verlag, 1996), so daß es keiner weiteren Erörterung bedarf.

Darüber hinaus betrifft die Erfindung estergruppenspaltende Zusammensetzungen, die ein erfindungsgemäßes estergruppenspaltendes Enzym und/oder ein entsprechendes synthetisches Protein mit gleicher Funktion und/oder Aminosäuresequenz sowie gegebenenfalls zusätzliche Enzyme, Stabilisatoren, geeignete oberflächenaktive Substanzen und/oder geeignete organische Lösungsmittel enthält.

Vorzugsweise handelt es sich bei den zusätzlichen Enzymen um Hydrolasen, insbesondere Esterasen, Proteasen, Cutinasen, Lipasen, Phospholipasen und Lysophospholipasen.

Besonders bevorzugt stammen diese Hydrolasen aus unter Pseudomonas sp., Rizomucor miehei, Candida cylindracea, Candida antartica, Aspergillus niger, Chromobacterium viscosum, Commamonas acidovorans, Rhizopus arrhizus und Rhizopus delamar ausgewählten Mikroorganismen. Besonders geeignet sind auch die in WO98/36086 (Bayer AG), auf die hier ausdrücklich Bezug genommen wird, offenbarten Mikroorganismen.

Die Erfindung gibt außerdem die Verwendung eines erfindungsgemäßen estergruppenspaltenden Enzyms oder eines synthetischen Proteins mit gleicher Funktion und/oder Aminosäuresequenz oder einer erfindungsgemäßen estergruppenspaltenden Zusammensetzung zum Abbau von Estergruppen enthaltenden niedermolekularen und/oder makromolekularen synthetischen oder natürlichen Verbindungen an.

Vorzugsweise handelt es sich bei den Estergruppen enthaltenden makromolekularen Verbindungen um aliphatische, cycloaliphatische, aliphatisch-aromatische, teilaromatische oder aromatische Polyester bzw. Copolyester, Polyesteramide, Polyestercarbonate oder Polyesterurethane, die kettenverlängert, verzweigt oder vernetzt sein können.

Die Estergruppen enthaltenden makromolekularen Verbindungen können beliebige Form haben und beispielsweise Copolymere, Mischungen bzw. Blends, Composits, Laminate oder Verklebungen mit anderen Werkstoffen bilden.

In Verfahren zum Abbau von Estergruppen enthaltenden niedermolekularen und/oder makromolekularen (polymeren) Verbindungen unter Verwendung des erfindungsgemäßen estergruppenspaltenden Enzyms (oder eines anhand der Aminosäuresequenz synthetisch hergestellten Enzyms) oder einer dieses enthaltenden Zusammensetzung können Auflösungsgeschwindigkeiten erreicht werden, die denen von bislang bekannten Systemen deutlich überlegen sind und eine technische Nutzung der enzymatischen Behandlung von Estergruppen enthaltenden Polymeren ermöglichen. Dies gilt insbesondere für aliphatisch-aromatische Copolyester und Polyester-Blends, die eine hohe wirtschaftliche Bedeutung haben.

Die Verwendung des erfindungsgemäßen estergruppenspaltenden Enzyms (oder eines anhand der Aminosäuresequenz synthetisch hergestellten Enzyms) oder einer diese enthaltenden Zusammensetzung zur Behandlung der oben bzw. im folgenden genannten Polymeren in technisch relevanten Formen, beispielsweise Folien, Spritzgußteile, Beschichtungen, Laminate, Schäumen, Partikel, Verklebungen, kann zur Erhöhung der Metabolisierungsgeschwindigkeit durch Mikroorganismen, zur Aufarbeitung von Produkten im Rahmen eines Recyclings (z.B. zum Lösen von Verklebungen oder Entfernen von Beschichtungen) zur Rückgewinnung von Polymerbausteinen aus bioabbaubaren Polymeren oder zur Oberflächenmodifizierung von Produkten aus Polyestern dienen.

Die Behandlung der Polymeren mit einer geeigneten Enzymformulierung, beispielsweise in Form eines rohen Kulturüberstandes von Thermomonospora fusca, der gegebenefalls konzentriert werden kann, eines gereinigten Enzyms oder eines synthetischen Enzyms oder einer diese enthaltenden Zusammensetzung, kann beispielsweise in wäßriger Lösung oder durch Auftragen der Enzymformulierung auf die Polymermaterialien erfolgen.

Niedermolekulare Esterverbindungen spielen als Additive in verschiedenen Polymeren eine Rolle. Auch solche Verbindungen lassen sich mit dem erfindungsgemäßen Enzym spalten.

Die durch das erfindungsgemäße Enzym (oder durch ein anhand der Aminosäuresequenz synthetisch hergestellten Enzyms) und/oder die diese enthaltende Zusammensetzung abbaubaren estergruppenhaltigen Polymeren umfassen neben den bereits oben genannten beispielsweise folgende:

Estergruppen enthaltende synthetische und natürliche Polymere, insbesondere Lignine, Lignocellulose, Cutin, Suberin, aliphatische Polyester, insbesondere die in WO98/36086 (Bayer AG), auf die hier ausdrücklich Bezug genommen wird, offenbarten, besonders bevorzugt Polycaprolacton, aromatische oder teilaromatische Copolyester, insbesondere die in WO98/36086 (Bayer AG) offenbarten, besonders bevorzugt Terephthalsäure enthaltende, ganz besonders bevorzugt Copolyester aus 1,4-Butandiol, Terephthalsäure und Adipinsäure (BTA), besonders bevorzugt mit einem Anteil von 30-70 Mol-% Terephthalsäure, Polyesteramide, insbesondere die in WO98/36086 (Bayer AG) offenbarten, Polymere, die Urethan- und Estergruppen enthalten, d. h. Polyesterurethane, und segmentierte Polyurethane.

Die Polyester können kettenverlängert, verzweigt oder vernetzt sein.

Besonders bevorzugte konkrete Polyester sind Poly(propylensuccinat), Poly(butylensuccinat), Poly(butylensuccinat-coethylensuccinat), ein Copolymer aus Bernsteinsäure/Adipinsäure/1,2-Ethandiol/1,4-Butandiol, Copolymere aus 1,4-Butandiol/Adipinsäure/Terephthalsäure.

Die durch das erfindungsgemäße Enzym (oder durch ein anhand der Aminosäuresequenz synthetisch hergestellten Enzyms) und/oder die diese enthaltende Zusammensetzung abbaubaren estergruppenhaltigen Polymeren können beispielsweise vorliegen als:
Copolymere oder Gemische (Blends) aus zwei oder mehreren der oben genannten Polymeren,
Composits oder Laminate aus zwei oder mehreren der oben genannten Polymeren oder deren Copolymeren oder Blends,
Composits, Laminate oder Verklebungen mit natürlichen oder modifizierten natürlichen polymeren Werkstoffen, insbesondere Stärke und/oder Cellulose (z. B. Papier),
Composits, Laminate oder Verklebungen mit anderen, nicht notwendigerweise bioabbaubaren Werkstoffen (z.B. Glas),

Polymerformulierungen, die übliche Füllmittel, Faserverstärkungen, Hilfsmittel, Stabilisatoren enthalten.

Die erfindungsgemäße Verwendung umfaßt die Behandlung von Polymeren in Form von Partikeln, Suspensionen, Emulsionen, Beschichtungen, Verklebungen, Filmen, Formkörpern, Fasern oder Vliesen, Geweben, Schäumen. Die Materialien können chemisch, thermisch oder mechanisch vorbehandelt oder unbehandelt eingesetzt werden.

Das Enzym wird beispielsweise in gepufferter Lösung oder in ungepufferter Lösung, gegebenenfalls unter Einstellung des pH-Wertes verwendet.

Die Anwendung erfolgt beispielsweise durch Einbringen von Estergruppen enthaltenden Substanzen in geeignete Enzymlösungen oder durch Aufbringen einer geeigneten Enzymformulierung auf entsprechende Substanzoberfächen.

Weitere Verwendungsmöglichkeiten des erfindungsgemäßen Enzyms betreffen die Behandlung der oben definierten Materialien zum Zweck der Vorbehandlung im Zuge einer Entsorgung, die Behandlung der Materialien zur Trennung von Produktkomponenten, die Behandlung der Materialien zum Zweck der Rückgewinnung einzelner oder aller Materialbausteine und die Behandlung von Materialien zum Zweck der Änderung von Oberflächeneigenschaften.

Die folgenden Beispiele dienen zur Veranschaulichung der Erfindung und sind nicht als Beschränkung aufzufassen.

### 1.Kultivierung von Thermomonospora fusca DSM 43793.

Ein steriler mit Alukappen verschließbarer Kulturkolben ohne Schikanen wird zwei Zentimeter hoch mit sterilem Medium (entsprechend DIN V 54900, Teil 2) gefüllt. In den Kolben werden 3 g/l eines aus 1,4-Butandiol, Terephthalsäureester und Adipinsäure synthetisierten Copolyesters gegeben und mit 1 Vol.-% des Inoculums aus einer Vorkultur von Thermomonospora fusca beimpft. Die Kultur wird 18 h bei 55°C auf einem Rundschüttler mit 120 Upm inkubiert.

Nach Abbruch der Kultur werden die Feststoffe mit 8000 x g bei 10°C 20 min abzentrifugiert. Der Überstand enthält das esterspaltende Enzym.

### 2. Abbau eines aliphatisch-aromatischen Copolyesters mit Thermomonospora fusca im Kulturüberstand.

Thermomonospora fusca DSM 43793 wird in einem Mineralsalzmedium (siehe Beispiel 1) 24,8 h bei 55°C kultiviert. 2 ml des organismenfreien Kulturüberstandes werden in eine Reagenzglas gegeben. Ein runder Polymerfilm (Durchmesser 0,9 cm) aus einem Copolyester aus Butandiol, Terephthalsäure und Adipinsäure (40 Mol.-% Terephthalsäure in der Säurekomponente) wird in den Kulturüberstand gegeben und 24 Stunden bei 55° C inkubiert. Der Gewichtsverlust des Films beträgt danach 2,575 mg/(cm² Oberfläche).

### 3. Isolierung des erfindungsgemäßen esterspaltenden Enzyms.

### Konzentrierung:

Der Kulturüberstand aus Beispiel 1 wird in einer Amicon-Ultrafiltrationskammer (Volumen: 50 ml, Filtrationsfläche: 47 mm²) unter einem Druck von 3 bar und einer Membran mit einem Cut- off von 10 kDa auf 5% des ursprünglichen Volumens konzentriert.

Die weiter Reinigung erfolgt mit Hilfe einer Standard-FPLC-Anlage "LCC-Plus" mit automatischer Äquilibrierung, Injektion und Elution (Pharmacia, Uppsala, Schweden). Das konzentrierte Protein im Kulturüberstand (2,1 mg) wird in einem ersten Schritt über eine Ionenaustauschersäule gereinigt.

### Parameter:

Säule: UNO-S1-Säule (Säulenvolumen 1,3 ml, BioRad, München)
Startpuffer: 20 mM Citratpuffer (pH 4,0)
Elution: (linearer Gradient) 1 M NaCl im Startpuffer
Flußrate: 2 ml/min

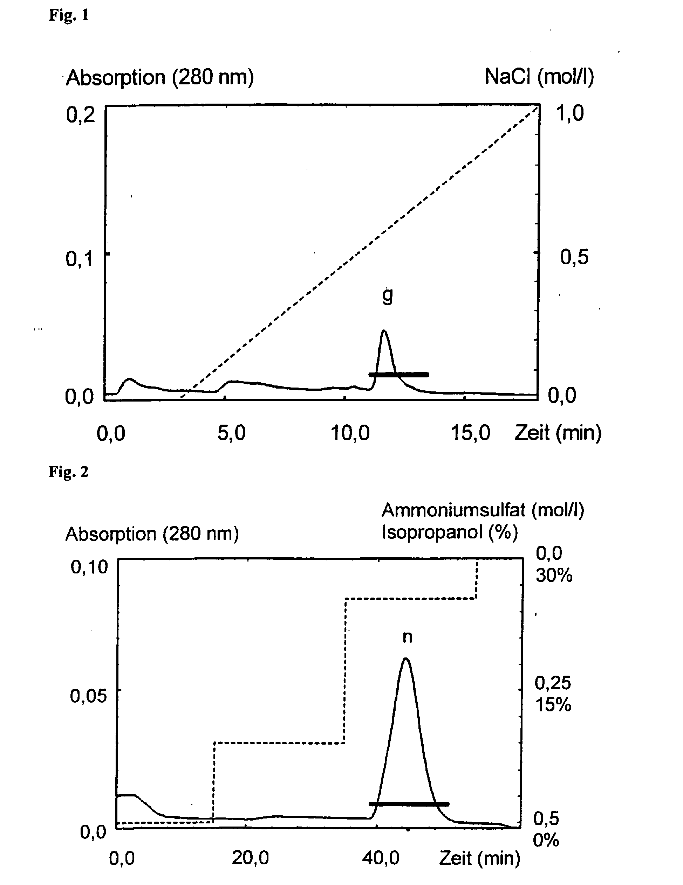

Figur 1 zeigt das Elutionsprofil, wobei der schwarze Balken die Fraktionen markiert, die estergruppenspaltende Aktivität aufweisen.

In einem zweiten Schritt werden durch Ionenaustauschchromatographie erhaltene und Aktivität aufweisende Fraktionen durch hydrophobe Interaktionschromatographie (HIC) weiter gereinigt.

116 µg Protein aus durch Ionenaustauschchromatographie erhaltenen Fraktionen werden auf eine Phenylsepharosesäule aufgetragen
Säule: Phenylsepharose-CL4B-Säule (Säulenvolumen: 1,14 ml, Pharmacia, Uppsala, Schweden)
Startpuffer: 0,5 M Ammoniumsulfat in 20 mM Phosphatpuffer (pH 7,1)
Elution: (Stufengradient) 30% Isopropanol in 20 mM Phosphatpuffer (pH 7,1)
Flußrate: 0,3 ml/min.

Figur 2 zeigt das Elutionsprofil, wobei der schwarze Balken die Fraktionen markiert, die estergruppenspaltende Aktivität aufweisen.

Der Kulturüberstand weist eine spezifische Aktivität von 3,3 U/mg auf. Nach der Ionenaustauschchromatographie wird eine spezifische Aktivität von 218 U/mg und nach der HIC eine von 360 U/mg erhalten.

### Charakterisierung des erfindungsgemäßen Enzyms.

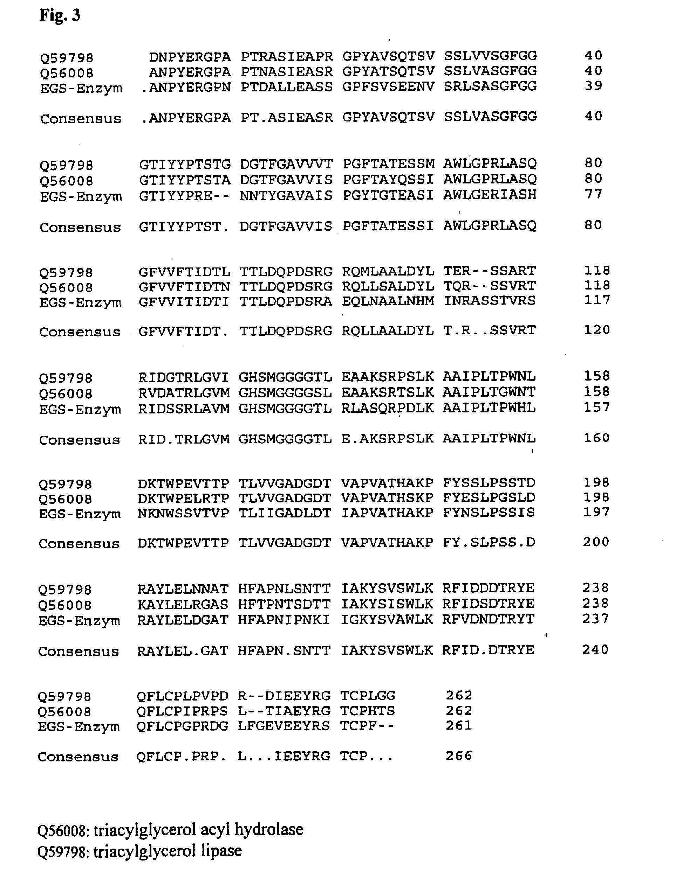

Figur 3 zeigt die Aminosäuresequenz des erfindungsgemäßen Enzyms und das "Alignement" zum Sequenzvergleich mit der Triacylglycerol-Lipase aus Streptomyces albus G und der Triacylglycerol-Acylhydrolase aus Streptomyces sp. M11. Das "Multiple Alignment" wurde mit dem Programm "PileUp" erstellt (Wisconsin Package, Version 9.1, Genetics Computer Group, Madison, WI, USA). Voneinander abweichende Aminosäuren an gleichen Positionen sind schattiert dargestellt. Die schwarz umrandete Box markiert eine hochkonservierte Aminosäuresequenz aus dem Bereich des aktiven Zentrums von Lipasen. Die Sequenzen der beiden Streptomyces-Stämme stammen aus der SP-TREMBL-Datenbank (Release 7.0, 08/1998): Q56008 (Streptomyces sp. M11), Q59798 (Streptomyces albus G).

Zur Aminosäuresequenzierung wurde das EGS-Enzym von den nach der Reinigung noch vorhandenen Fremdproteinen isoliert. Dies erfolgte durch Auftrennung der Proteine mittels präparativer SDS-Gelelektrophorese und Übertragung auf eine PVDF-Membran durch Western-Blotting. Nach der Färbung der Proteinbanden wurde die Bande des Enzyms aus der Membran ausgeschnitten und sequenziert.

Zur Bestimmung der Gesamtsequenz wurde das Enzym mit Trypsin und GIuC verdaut. Die Trennung der entstandenen Peptide erfolgte durch HPLC ("reversed phase"). Die N-terminale Sequenz und die Peptidfraktionen aus der Verdauung der BTA-Hydrolase wurden über einen "Edman-Abbau" in einem "Applied Biosystems 473A Sequencer" ("gas-phase-mode") oder in einem "494A Procise HT Sequencer" ("gas-phase"- und "pulsed-liquid-mode") mit Standardprogrammen des Herstellers analysiert.

Durch Sequenzüberlappung und durch Vergleich der Teilsequenzen des EGS-Enzyms mit den Aminosäuresequenzen zweier bekannter Streptomyces-Lipasen wurde die Gesamtsequenz des Enzyms bestimmt.

### 4. Abbau von Estergruppen enthaltenen Polymeren mit dem erfindungsgemäßen estergruppenspaltenden Enzym.

Unter sterilen Bedingungen wurde in Reagenzgläsern je ein Polymerfllm (d = 0,9 cm) mit 1 ml der gereinigten Enzymlösung (25 µg Enzym in 20 mM Phosphatpuffer, pH 7,1) versetzt. Die Reagenzgläser wurden 17 h bei 55°C inkubiert. Der Gewichtsverlust der Polymerfilme diente als Maß für die Enzymaktivität.

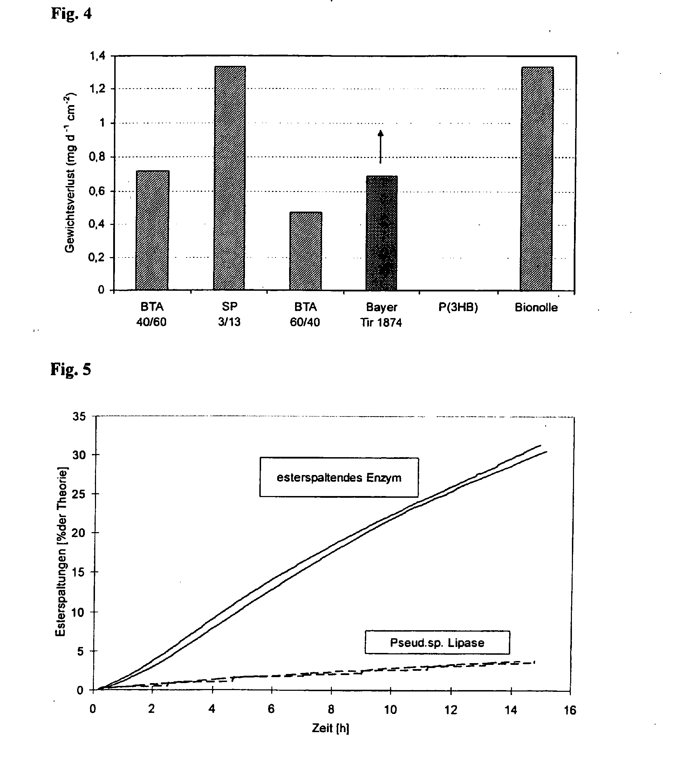

Neben dem aliphatisch-aromatischen Copolyestern BTA4O:60 (40 mol% Terephthalsäure in der Säurekomponente) und BTA 60:40 (60 mol% Terephthalsäure in der Säurekomponente) wird ein aliphatischer Polyester SP3:13 (aus 1,3-Propandiol und Brassylsäure synthetisiert) sowie die kommerziellen Estergruppen enthaltenden Polymere Bayer Tir 1874 (Polyesteramid der Firma Bayer AG), Bionolle (aliphatischer Polyester der Firma Showa Highpolymers) sowie der natürliche bakterielle Polyester P(3HB) abgebaut. Gegenüber P(3HB) weist das estergruppenspaltende Enzym keine erkennbare Aktivität auf. Bayer Tir 1874 war zum Zeitpunkt der Probenahme schon vollständig solübilisiert und die angegebene Aktivität stellt einen Minimalwert dar. Die Ergebnisse sind in Figur 4 dargestellt.

### 5. Vergleich des erfindungsgemäßen esterspaltenden Enzyms mit der Lipase aus Pseudomonas sp.

In 6 ml physiologischer Kochsalzlösung (pH 7,0) werden jeweils Filme aus BTA40:60 gegeben. Zu der Lösung werden jeweils 50 µg des jeweiligen Enzyms (erfindunggemäßes esterspaltendes Enzym bzw. Lipase aus Pseudomonas sp. von SIGMA Chemical Co., EC 3.1.1.3) gegeben. Der Ansatz wird bei der jeweiligen optimalen Temperatur der Enzyme inkubiert. Der Fortschritt des Abbaus wird durch Titration der gebildeten freien Säuren mit 0,1 M NaOH verfolgt. Das Ergebnis ist in Figur 5 dargestellt.

Im Vergleich zur Pseudomonas-sp.-Lipase kann mit dem erfindungsgemäßen Enzym eine wesentlich höhere Hydrolysegeschwindigkeit erreicht werden.

### 6. Spaltung von Triglyceriden mit dem erfindungsgemäßen esterspaltenden Enzym und mit der Lipase von Pseudomonas sp.

0,5 ml der jeweiligen Triglyceride werden mit 5 ml einer Emulsionslösung (4,475 g NaCl, 0,103 g KH₂PO₄ in einem Gemisch aus 75 ml destilliertem Wasser und 135 ml Glycerin (99,5%) gelöst, mit 1,5 g Gummi-Arabicum versetzt und mit destilliertem Wasser auf 250 ml aufgefüllt) und mit 4,5 ml destilliertem Wasser versetzt.

Die Substratlösung wird direkt vor Beginn des Enzymtestes angesetzt und mit Hilfe eines Ultraturrax' 1 min bei 13500 Upm homogenisiert.

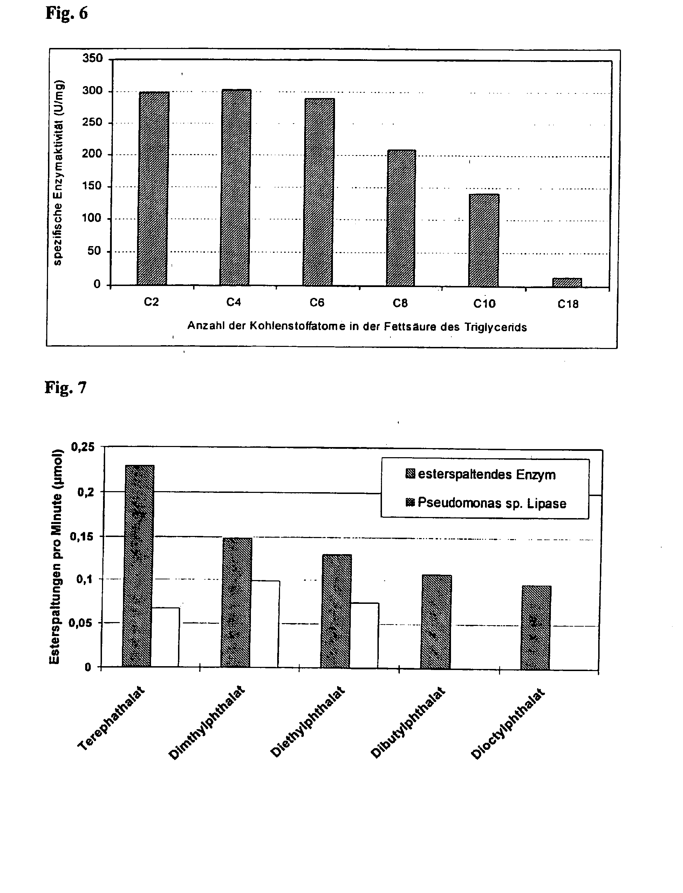

Danach wird die Substratlösung mit der Enzymlösung versetzt (20 µg Enzym pro 6 ml Substratlösung), der pH-Wert auf pH 7,1 eingestellt und die Esterspaltungen durch Titration mit 0,1 M NaOH verfolgt. In Figur 6 sind die Ergebnisse für Triglyceride mit verschiedener Anzahl an C-Atomen in der Fettsäurekomponente dargestellt.

Es kann ein breites Spektrum an Fettsäuren gespalten werden.

### 7. Spaltung von Phthalsäureestern mit dem erfindungsgemäßen esterspaltenden Enzym und mit der Lipase von Pseudomonas sp.

Die Versuchsansätze entsprechen denen von Beispiel 6. Anstelle der Triglyceride werden Phthalsäureester mit unterschiedlichen Alkoholkomponenten eingesetzt. Während die Lipase aus Pseudomonas sp. nur den Dimethyl- und Diethylester spalten kann, hydrolysiert das erfindungsgemäße Enzym auch die Ester mit längerkettigen Alkoholen. Die Hydrolysegeschwindigkeiten sind höher als die der Pseudomonas-sp.-Lipase. Die Ergebnisse sind in Figur 7 dargestellt.

## Patentansprüche

1. Estergruppen von Polyestern spaltendes Enzym, das erhältlich ist, indem der Mikroorganismus Thermomonospora fusca in einem geeigneten Nährmedium in Anwesenheit eines Induktors kultiviert wird, aus dem Nährmedium ein Überstand mit einem Gehalt an einem Estergruppen von Polyestern spaltenden Enzym gewonnen wird, und das Enzym mit üblichen biochemischen Reinigungsmethoden gereinigt wird, wobei das Enzym durch folgende Parameter **gekennzeichnet** ist:
Molmasse: 27400 D (durch SDS-Gelelektrophorese bestimmt) bzw. 28200 D (aus der Aminosäuresequenz berechnet)
Temperaturoptimum/-bereich: 65°C (30-80°C),
Temperaturstabilität 70°C/30 min,
pH-Optimum/-bereich: 6-7 (4- >8),
Isoelektrischen Punkt:6,4.

2. Estergruppenspaltendes Enzym nach Anspruch 1, wobei es sich bei dem Mikroorganismus um einen Thermomonospora-fusca-Stamm handelt, der bei der Deutschen Sammlung für Mikroorganismen unter der Zugangsnummer DSM 43793 hinterlegt ist.

3. Estergruppenspaltendes Enzym nach einem der vorherigen Ansprüche, wobei das Enzym aus dem Nährmedium isoliert wird, indem aus dem Nährmedium ein enzymhaltiger Kulturüberstand gewonnen wird, der gegebenenfalls konzentriert werden kann, und durch Chromatographie, insbesondere durch Ionenaustausch- und/oder hydrophobe Interaktionschromatographie, das Enzym gereinigt wird.

4. Estergruppenspaltendes Enzym nach einem der vorherigen Ansprüche, **gekennzeichnet durch** folgende Aminosäuresequenz: oder
**durch** Substituition, Insertion oder Deletion von Aminosäuren entstandene Mutanten, die Estergruppen von Polyestern spalten (isofunktionelle Enzyme).

5. Synthetisches estergruppenspaltendes Protein mit der Aminosäuresequenz des estergruppenspaltendes Enzyms nach Anspruch 4.

6. Polyklonaler Antikörper, der spezifisch gegen ein esterspaltendes Enzym nach einem der Ansprüche 1 bis 4 oder gegen ein synthetisches Protein nach Anspruch 5 gerichtet ist.

7. Monoklonaler Antikörper, der spezifisch gegen ein esterspaltendes Enzym nach einem der Ansprüche 1 bis 4 oder gegen ein synthetisches Protein nach Anspruch 5 gerichtet ist.

8. Hybridomzelle, die einen monoklonalen Antikörper nach Anspruch 7 bildet.

9. Estergruppenspaltende Zusammensetzung, die ein estergruppenspaltendes Enzym nach einem der Ansprüche 1 bis 4 und/oder ein synthetisches Peptid oder Protein nach Anspruch 5 sowie gegebenenfalls zusätzliche Enzyme, Stabilisatoren, geeignete oberflächenaktive Substanzen und/oder geeignete organische Lösungsmittel enthält.

10. Estergruppenspaltende Zusammensetzung nach Anspruch 9, wobei die zusätzlichen Enzyme Hydrolasen, insbesondere Esterasen, Proteasen, Cutinasen, Lipasen, Phosphoplipasen und Lysophosphoplipasen, sind.

11. Estergruppenspaltende Zusammensetzung nach Anspruch 10, wobei die Hydrolasen aus unter Pseudomonas sp., Rizomucor miehei, Candida cylindracea, Candida antartica, Asperigillus niger, Chromobacterium viscosum, Commamonas acidovorans, Rhizopus arrhizus und Rhizopus delamar ausgewählten Mikroorganismen stammen.

12. Verwendung eines estergruppenspaltenden Enzyms nach einem der Ansprüche 1 bis 4 oder eines synthetischen Proteins nach Anspruch 5 oder einer estergruppenspaltenden Zusammensetzung nach einem der Ansprüche 9 bis 11 zum Abbau von Estergruppen enthaltenden niedermolekularen und/oder makromolekularen synthetischen oder natürlichen Verbindungen.

13. Verwendung nach Anspruch 12, wobei es sich bei den Estergruppen enthaltenden makromolekularen Verbindungen um aliphatische, cycloaliphatische, aliphatisch-aromatische, teilaromatische oder aromatische Polyester bzw. Copolyester, Polyesteramide, Polyestercarbonate oder Polyesterurethane handelt, die kettenverlängert, verzweigt oder vernetzt sein können.

14. Verwendung nach Anspruch 13, wobei die Estergruppen enthaltenden makromolekularen Verbindungen Copolymere, Mischungen bzw. Blends, Composits, Laminate oder Verklebungen mit anderen Werkstoffen bilden.

15. Verfahren zur Herstellung eines Estergruppen von Polyestern spaltenden Enzyms, bei dem der Mikroorganismus Thermomonospora fusca in einem geeigneten Nährmedium in Anwesenheit eines Induktors kultiviert wird, aus dem Nährmedium ein Überstand mit einem Gehalt an einem Estergruppen von Polyestern spaltenden Enzym gewonnen wird, und das Enzym mit üblichen biochemischen Reinigungsmethoden gereinigt wird, wobei das Enzym durch folgende Parameter **gekennzeichnet** ist:
Molmasse: 27400 D (durch SDS-Gelelektrophorese bestimmt) bzw. 28200 D (aus der Aminosäuresequenz berechnet)
Temperaturoptimum/-bereich: 65°C (30-80°C),
Temperaturstabilität 70°C/30 min,
pH-Optimum/-bereich: 6-7 (4- >8),
Isoelektrischen punkt:6,4.

## Claims

1. Enzyme that cleaves ester groups of polyesters, which enzyme is obtainable by culturing the microorganism Thermomonospora fusca in a suitable nutrient medium in the presence of an inducer, obtaining from the nutrient medium a supernatant that contains an enzyme that cleaves ester groups of polyesters, and purifying the enzyme by customary biochemical methods of purification, wherein the enzyme is **characterised by** the following parameters:
molecular weight: 27400 D (determined by SDS gel electrophoresis) or 28200 D (calculated on the basis of the amino acid sequence),
temperature optimum/range: 65°C (30-80°C),
temperature stability: 70°C/30 min,
pH optimum/range: 6-7 (4- >8),
isoelectric point: 6.4.

2. Ester-group-cleaving enzyme according to claim 1, the microorganism being a Thermomonospora fusca strain that has been deposited with the Deutsche Sammlung für Mikroorganismen [German Collection of Microroganisms] under the number DSM 43793.

3. Ester-group-cleaving enzyme according to either of the preceding claims, the enzyme being isolated from the nutrient medium by obtaining an enzyme-containing culture supernatant from the nutrient medium, which supernatant may optionally be concentrated, and purifying the enzyme by chromatography, especially by ion exchange chromatography and/or hydrophobic interaction chromatography.

4. Ester-group-cleaving enzyme according to any one of the preceding claims, **characterised by** the following amino acid sequence: or
mutatants resulting from substitution, insertion or deletion of amino acids, which mutations cleave ester groups of polyesters (isofunctional enzymes).

5. Synthetic ester-group-cleaving protein having the amino acid sequence of the ester-group-cleaving enzyme according to claim 4.

6. Polyclonal antibody directed specifically against an ester-cleaving enzyme according to any one of claims 1 to 4 or against a synthetic protein according to claim 5.

7. Monoclonal antibody directed specifically against an ester-cleaving enzyme according to any one of claims 1 to 4 or against a synthetic protein according to claim 5.

8. Hybridoma cell that produces a monoclonal antibody according to claim 7.

9. Ester-group-cleaving composition that comprises an ester-group-cleaving enzyme according to any one of claims 1 to 4 and/or a synthetic peptide or protein according to claim 5 and optionally additional enzymes, stabilisers, suitable surface-active substances and/or suitable organic solvents.

10. Ester-group-cleaving composition according to claim 9, wherein the additional enzymes are hydrolases, especially esterases, proteases, cutinases, lipases, phospholipases and lysophospholipases.

11. Ester-group-cleaving composition according to claim 10, wherein the hydrolases originate from microorganisms selected from Pseudomonas sp., Rizomucor miehei, Candida cylindracea, Candida antartica, Aspergillus niger, Chromobacterium viscosum, Commamonas acidovorans, Rhizopus arrhizus and Rhizopus delamar.

12. Use of an ester-group-cleaving enzyme according to any one of claims 1 to 4 or of a synthetic protein according to claim 5 or of an ester-group-cleaving composition according to any one of claims 9 to 11 for the degradation of ester-group-containing low molecular weight and/or macromolecular synthetic or natural compounds.

13. Use according to claim 12, wherein the ester-group-containing macromolecular compounds are aliphatic, cycloaliphatic, aliphatic-aromatic, partially aromatic or aromatic polyesters or copolyesters, polyesteramides, polyestercarbonates or polyesterurethanes, the chain of which may be extended, and which may be branched or crosslinked.

14. Use according to claim 13, wherein the ester-group-containing macromolecular compounds form copolymers, mixtures or blends, composites, laminates or adhesive bonds with other materials.

15. Method for the preparation of an enzyme that cleaves ester groups of polyesters, wherein the microorganism Thermomonospora fusca is cultured in a suitable nutrient medium in the presence of an inducer, a supernatant containing an enzyme that cleaves ester groups of polyesters is obtained from the nutrient medium, and the enzyme is purified by customary biochemical methods of purification, wherein the enzyme is **characterised by** the following parameters:
molecular weight: 27400 D (determined by SDS gel electrophoresis) or 28200 D (calculated on the basis of the amino acid sequence),
temperature optimum/range: 65°C (30-80°C),
temperature stability: 70°C/30 min,
pH optimum/range: 6-7 (4- >8),
isoelectric point: 6.4.

## Revendications

1. Enzyme de clivage de groupes esters de polyesters, laquelle peut être obtenue en cultivant le microorganisme *Thermomonospora fusca* dans un milieu nutritif approprié en présence d'un inducteur, en obtenant à partir du milieu nutritif un surnageant contenant une enzyme de clivage de groupes esters de polyesters et en purifiant l'enzyme avec des procédés de purification biochimiques habituels, l'enzyme étant **caractérisée par** les paramètres suivants :
masse moléculaire : 27 400 D (déterminée par électrophorèse sur gel SDS) ou de 28 200 D (calculée en se basant sur la séquence d'acides aminés),
température optimale/plage de température : 65 °C (30 à 80 °C),
stabilité de la température : 70 °C/30 min,
pH optimal/plage du pH : 6 à 7 (4 à supérieur à 8),
point isoélectrique : 6,4.

2. Enzyme de clivage de groupes esters selon la revendication 1, dans laquelle le microorganisme est une souche de *Thermomonospora fusca* qui a été déposée auprès de la Deutschen Sammlung für Mikroorganismen sous le numéro DSM 43793.

3. Enzyme de clivage de groupes esters selon l'une des revendications précédentes, l'enzyme étant isolée du milieu nutritif en obtenant à partir du milieu nutritif un surnageant de culture contenant l'enzyme, lequel surnageant peut être concentré le cas échéant, et l'enzyme étant purifiée par chromatographie, notamment par chromatographie échangeuse d'ions et/ou par chromatographie d'interaction hydrophobe.

4. Enzyme de clivage de groupes esters selon l'une des revendications précédentes, **caractérisée par** la séquence d'acides aminés suivante : ou
par la substitution, l'insertion ou la délétion de mutants provenant d'acides aminés, qui clive les groupes esters de polyesters (enzymes isofonctionnelles).

5. Protéine synthétique de clivage de groupes esters avec la séquence d'acides aminés de l'enzyme de clivage de groupes esters selon la revendication 4.

6. Anticorps polyclonal dirigé spécifiquement contre une enzyme de clivage d'esters selon l'une des revendications 1 à 4 ou contre une protéine synthétique selon la revendication 5.

7. Anticorps monoclonal dirigé spécifiquement contre une enzyme de clivage d'esters selon l'une des revendications 1 à 4 ou contre une protéine synthétique selon la revendication 5.

8. Cellule d'hybridome qui forme un anticorps monoclonal selon la revendication 7.

9. Composition de clivage de groupes esters qui contient une enzyme de clivage de groupes esters selon l'une des revendications 1 à 4 et/ou un peptide ou une protéine synthétique selon la revendication 5 ainsi que, le cas échéant, des enzymes supplémentaires, des stabilisants, des substances tensioactives appropriées et/ou des solvants organiques appropriés.

10. Composition de clivage de groupes esters selon la revendication 9, dans laquelle les enzymes supplémentaires sont les hydrolases, notamment les estérases, les protéases, les cutinases, les lipases, les phospholipases et les lysophospholipases.

11. Composition de clivage de groupes esters selon la revendication 10, dans laquelle les hydrolases proviennent de microorganismes choisis parmi *Pseudomonas sp., Rizomucor miehei, Candida cylindracea, Candida antartica, Aspergillus niger, Chromobacterium viscosum, Commamonas acidovorans, Rhizopus arrhizus* et *Rhizopus delamar.*

12. Utilisation d'une enzyme de clivage de groupes esters selon l'une des revendications 1 à 4 ou d'une protéine synthétique selon la revendication 5 ou d'une composition de clivage de groupes esters selon l'une des revendications 9 à 11 pour la décomposition de composés naturels ou synthétiques de bas poids moléculaires et/ou macromoléculaires contenant des groupes esters.

13. Utilisation selon la revendication 12, dans laquelle les composés macromoléculaires contenant des groupes esters sont des polyesters ou copolyesters, des polyesteramides, des polyestercarbonates ou des polyesteruréthanes aliphatiques, cycloaliphatiques, aliphatiques-aromatiques, partiellement aromatiques ou aromatiques dont la chaîne peut être rallongée et qui peuvent être ramifiés ou réticulés.

14. Utilisation selon la revendication 13, dans laquelle les composés macromoléculaires contenant des groupes esters forment des copolymères, des mélanges, des composites, des stratifiés ou des liaisons adhésives avec d'autres substances.

15. Procédé pour préparer une enzyme de clivage de groupes esters de polyesters, dans lequel le microorganisme *Thermomonospora fusca* est cultivé dans un milieu nutritif approprié en présence d'un inducteur, un surnageant contenant une enzyme de clivage de groupes esters de polyesters est obtenu à partir du milieu nutritif et l'enzyme est purifiée avec des procédés de purification biochimiques habituels, l'enzyme étant **caractérisée par** les paramètres suivants :
masse moléculaire : 27 400 D (déterminée par électrophorèse sur gel SDS) ou de 28 200 D (calculée en se basant sur la séquence d'acides aminés),
température optimale/plage de température : 65 °C (30 à 80 °C),
stabilité de la température : 70 °C/30 min,
pH optimal/plage du pH : 6 à 7 (4 à supérieur à 8),
point isoélectrique : 6,4.
